# EUROPEAN PATENT SPECIFICATION

(11) **EP 1 974 675 B1**
(45) Date of publication and mention of the grant of the patent: **16.12.2009**
(21) Application number: 08002573.7
(22) Date of filing: 12.02.2008
(51) Int. Cl.: A61B 10/06, A61B 10/04, A61B 17/28

(54) **Endoscopic treatment tool**
Endoskopisches Behandlungswerkzeug
Outil de traitement endoscopique

(30) Priority: 26.03.2007 JP 2007078266
(43) Date of publication of application: 01.10.2008
(73) Proprietor: Fujinon Corporation, Saitama-shi, Saitama (JP)
(72) Inventor: Akahane, Hidefumi, Saitama-shi Saitama (JP)
(74) Representative: Klunker . Schmitt-Nilson . Hirsch

(56) References cited:
- US-A- 4 945 920
- US-A- 5 904 647
- US-A- 6 015 381
- US-B1- 6 299 630
- US-B1- 6 443 909

## Description

### Background of the Invention

### 1. Field of the Invention

The present invention relates to an endoscopic treatment tool that is insertedusing an endoscopic treatment tool inserting channel as a guide section.

### 2. Description of the Related Art

A treatment tool to be inserted into a body cavity of a subject so as to perform treatments, such as the cure of a diseased part and the collection of tissue cells, is inserted thereinto using an endoscopic treatment tool inserting channel as a guide section. That is, an operation member, such as a forceps, is provided at an end of a flexible cord. Also, an operation member operating section is mounted in a base end portion of the flexible cord. An operating wire inserted in the flexible cord connects between the operation member and the operating section.

The operation member to be provided at an end of a forceps includes that, such as a forceps, of the type to be opened and closed, and that, such as a high-frequency snare, of the type to be pushed and pulled. In either case, an operating wire is inserted in a flexible cord. This operating wire is pushed and pulled by an operating section. For example, an operating wire is provided by being connected to a link mechanism that is coupled to a pair of forceps portions. Alternatively, in a high frequency snare, an operating wire is configured so that a snare wire provided at an end thereof goes in and out of a flexible cord, and that a high frequency current can be supplied to the snare wire.

Although the flexible cord is constituted by inserting the operating wire therein, it is necessary in the forceps and the high frequency snare to perform the direction control of the operation member. Thus, in the case of a treatment tool requiring the direction control of an operation member thereof, a flexible cord is configured not only to have flexibility in a bending direction but also to transmit torque to a tip end portion thereof when an operator twists an operating member or a base end portion of the flexible cord at the side of his hand in a state in which the flexible cord is inserted in a treatment tool inserting channel. The treatment tool capable of turning the flexible cord inserted in the treatment tool inserting channel is disclosed in JP-A-10-192286 and JP-A-2000-229084.

The treatment tool disclosed in JP-A-10-192286 is constituted by putting a mesh tube, which is made by netting thin stainless steel wires into between synthetic resin tubes and by performing thermal adhesion of the mesh tube to the synthetic resin tubes. That is, the synthetic resin tubes are respectively attached to the inner surface and the outer surface of the mesh tube. Then, the synthetic resin tubes are softened by making heat act thereon so as to soak the synthetic resin of the synthetic resin tubes into the mesh of the mesh tube. Consequently, the flexible cord has flexibility in a bending direction and can transmit torque in a treatment tool inserting channel.

In the treatment tool disclosed in JP-A-2000-229084, a flexible cord is constituted by dual closely-wound coils that are obtained by winding the closely-wound coils in opposite directions, respectively. Each of the closely wound coils is formed of a flexible metal wire. Thus, the flexible cord has flexibility in a bending wire. Also, even in the case of turning the flexible cords in a clockwise or counterclockwise direction, one of the closely wound coils is brought into a closely contacted state. Consequently, torque is transmitted to a tip end of the flexible cord.

Meanwhile, as described above, the flexible cord of the endoscopic treatment tool has flexibility in a bending direction. In addition, the flexible cord of the endoscopic treatment tool has the property that when the base end side part thereof is twisted, torque is surely transmitted to the tip end thereof. However, a path for the operating wire, which is formed in the flexible cord, should be assured. Also, it is necessary to reduce the diameter of the operating wire.

The treatment tool disclosed in JP-A-10-192286 has favorable torque transmissibility. However, this treatment tool has a problem in that the treatment tool has poor buckling resistance. That is, when a force acting in a bending direction is acted on the flexible cord, this cord is easilybuckled. Therefore, whenabendableportion provided in the vicinity of an end of an insertion portion is bent at an angle of 180 degrees or more in a state in which the treatment tool is inserted in the insertion portion of the endoscope, the flexible cord may be buckled due to a pressing force acting on the flexible cord. Thus, when the buckling of the flexible cord occurs, it is difficult to push and pull the operating wire inserted therein. In a serious case, it may be impossible to push and pull the operating wire.

On the other hand, in a case where the flexible cord is formed of dual closely-wound coils as disclosed in JP-A-2000-229084, the treatment tool has favorable buckling resistance and shape retainability. However, to that end, the diameter of the closely-wound coil should be increased to some extent. Thus, the outside diameter of the flexible cord is large. In addition, the flexibility thereof in a bending direction is reduced. Moreover, even the closely-wound coil cannot be wound in a completely contacted state. Consequently, a certain degree of play is provided when the flexible cord is turned. Accordingly, this treatment tool has a problem in that the torque transmissibility thereof is poor.

US 4,945,920 discloses a biopsy forceps comprising a handle portion, an elongate tubular torqueable and formable body assembly and a forceps assembly. The tubular body assembly includes an insertion path component in the form of a coil spring guide wire which extends between the handle portion and the forceps assembly. A first tubing over the proximal portion of the insertion path component extends from the handle portion towards the forceps assembly, this first tubing being torque resistant. A second tubing of formable material is received over and tightly engages the distal portion of the insertion path component, thereby imparting high formability to the distal portion of the insertion path component. The second tubing extends rearwardly from the forceps assembly to the distal end of the first tubing and overlaps the distal end of the first tubing.

### Summary of the Invention

The invention is accomplished in view of the aforementioned problems. An object of the invention is to provide an endoscopic treatment tool having a flexible cord that has favorable flexibility in a bending direction, sufficient buckling resistance, and excellent flexibility.

To achieve the aforementioned object, according to the invention, there is provided an endoscopic treatment tool configured to be inserted in an endoscopic-treatment-tool inserting channel, the endoscopic treatment tool comprising: a flexible cord; an operation portion provided at a tip end of the flexible cord; an operating wire, inserted in the flexible cord, that operates the operation portion; and an operating section, provided at a base end portion of the flexible cord, that operates the operating wire, wherein the flexible cord comprises a double-tube-like member including an insertion path component and a torque transmitting sleeve, the insertion path component includes a closely wound coil, and the torque transmitting sleeve includes a resin tube in which a mesh tube is embedded; and the insertion path component is provided in the torque transmitting sleeve by being inserted into the torque transmitting sleeve so as to be able to perform a relative sliding motion with respect with the torque transmitting sleeve, and the operating wire is inserted in the insertion path component.

Wherein the flexible cord comprises the double tube-like member over its entire length and wherein the insertion path component and the torque transmitting sleeve are in a non-fixed state in a lengthwise direction between the tip end and the base end of the flexible cord.

### Brief Description of the Drawings

FIG. 1 is a diagram illustrating the configuration of the entire forceps serving as an example of a treatment tool according to an embodiment of the invention;
FIG. 2 is an explanatory diagram illustrating a forceps operation portion provided at an end of the forceps;
FIG. 3 is an explanatory diagram illustrating the configuration of a flexible cord;
FIG. 4 is a cross-sectional diagram illustrating the flexible cord; and
FIG. 5 is an explanatory diagram illustrating a state in which the forceps is mounted in a treatment tool inserting channel of an endoscope.

### Detailed Description of the Invention

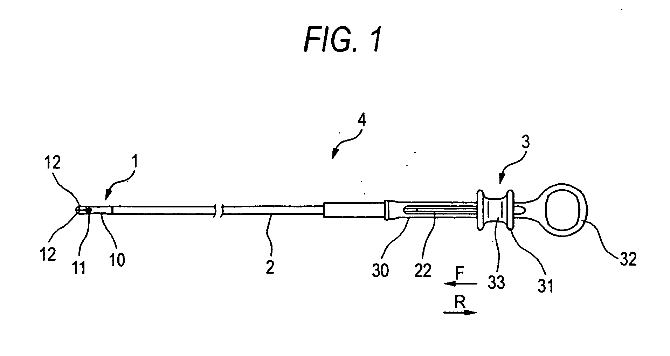

Hereinafter, an embodiment of the invention is described with reference to the accompanying drawings. Referring first to FIG. 1, the configuration of the entire treatment tool is described below. A forceps is, for example, a biopsy forceps, whose operation portion is shaped like a cup, or a straight grasping forceps shaped like a gong. Hereunder, a biopsy forceps is described by way of example. In FIG. 1, reference numeral 1 designates a forceps operation portion. Reference numeral 2 denotes a flexible cord. Reference numeral 3 represents an operationmember. A forceps 4 serving as an endoscopic treatment tool includes these components.

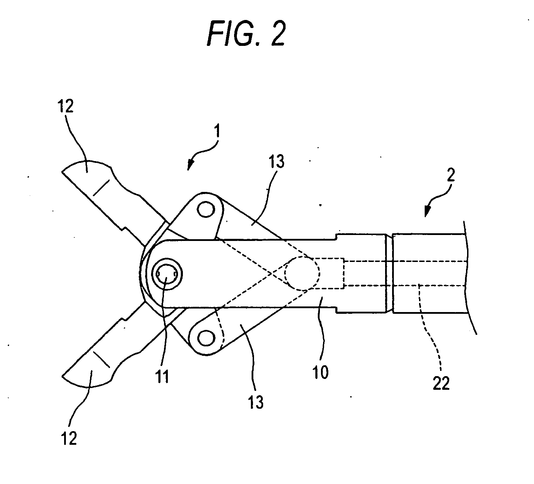

As illustrated in FIG. 2, a support shaft 11 is provided in a mounting part 10 of the forceps operation portion 1. Paired cup-like forceps portions 12, 12 are provided turnably around the support shaft 11. Then, a link mechanism 13 is provided so as to open and close the forceps portions 12, 12. The forceps portions 12, 12 are opened and closed by moving the link mechanism 13 back and forth.

The operating member 3 includes a slider 31 mounted on the shaft portion 30 movably in the direction of an axis line of the shaft portion 30. A finger engagement portion 32 is provided consecutively with an end part of the shaft portion 30. The slider 31 has an annular-groove-shaped finger engagement portion 33.

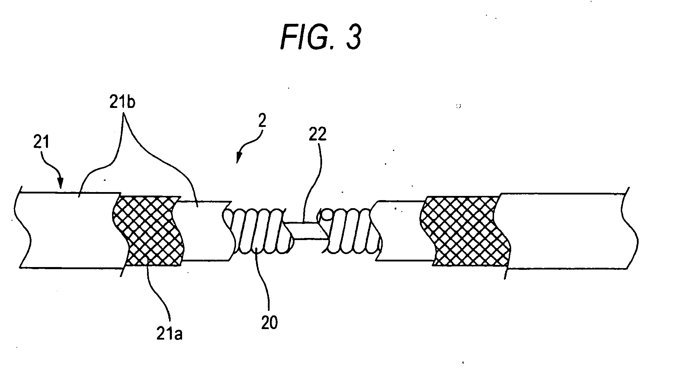

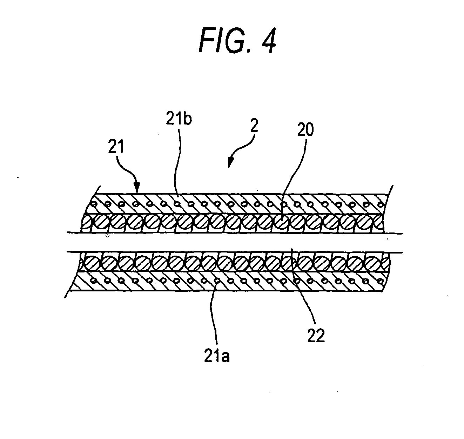

As illustrated in FIGS. 3 and 4, the flexible cord 2 includes a double tube having an insertion path component 20 and a torque transmitting sleeve 21. Both leading end portions of the insertion path component 20 and the torque transmitting sleeve 21 are fixed to the mounting part 10 of the forceps operation portion 1. Both base end portions of theinsertion path component 20 and the torque transmitting sleeve 21. are fixed to the shaft portion 30 of the operating member 3. On the other hand, the tip end of the operating wire 22 is coupled to the link mechanism 13. The base end portion of the operating wire 22 is coupled to the slider 31.

With the aforementioned configuration, when the slider 31 of the operating member 3 is slide-moved along the shaft portion 30 in the direction of arrow F shown in FIG. 1, the link mechanism 13, to which the tip end of the operating wire 22 is coupled, is pushed out, so that the forceps portions 12, 12 are opened. When the slider 31 is slide-moved in the direction of arrow R shown in FIG. 1, the operating wire 22 is displaced in the same direction, so that the link mechanism 13 is drawn toward the base end side, and that the forceps portions 12, 12 are closed. Thus, first, a body tissue is placed between the forceps portions 12, 12 in a state in which the forceps portions 12, 12 are opened. Then, treatments, such as the cutting and collection of the body tissue, can be performed by closing the forceps portions 12, 12.

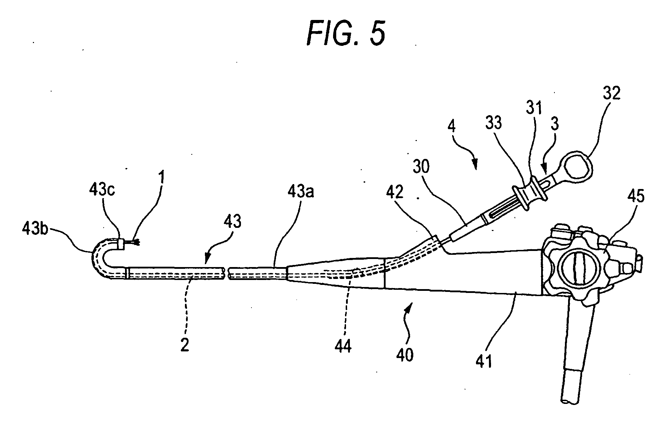

The forceps 4 serving as an endoscopic treatment tool is not singly inserted into a body cavity. As illustrated in FIG. 5, the forceps 4 is led into a body cavity by being inserted into an endoscope 40. A treatment tool introducing portion 42 is provided in a main body operating portion 41 in the endoscope 40. A treatment tool inserting channel 44 is provided to extend from the treatment tool introducing portion 42 to the tip end of the insertion portion 43. Accordingly, the treatment tool is inserted in the treatment tool inserting channel 44.

In the endoscope 40, a part of a predetermined length, which extends from the connection portion of the insertion portion 43 to the main body operating portion 41 is a soft portion 43a adapted to bend in a given direction along an insertion path . A curved portion 43b is provided consecutively with the soft portion 43a. Additionally, a rigid tip end portion 43c is provided consecutively with the curved portion 43b. A treatment tool leading-out portion for leading the treatment tool into a body cavity, together with an endoscopic observation section including an illumination portion and an observation portion, is opened in the rigid tip end portion 43c.

Accordingly, the flexible cord 2 of the forceps 4 has flexibility in a bending direction. The curved portion 43b of the insertion portion 43 is bent by a bending operation member 45. As illustrated in FIG. 5, the curved portion 43b is bent by an angle being equal to or larger than, for example, 180 degrees. Because the length of the curved portion 43b is short, the curvature radius thereof is extremely small. Thus, it is necessary to structure the forceps 4 as not to buckle even in a state in which the curved portion 43b is bent at maximum, as illustrated in FIG. 5. Also, a direction control operation should be achieved so that a body tissue can be held by the forceps portion 12, 12 of the forceps operation portion 1 in the treatment tool introducing portion 42.

To meet the aforementioned demands, the flexible cord 2 has a double tube structure including the insertion path component 20 and the torque transmitting sleeve 21, as illustrated in FIGS. 3 and 4. The insertion path component 20 serving as an inner layer includes a closely-wound coil, that is, a member obtained by closely winding a metal wire like a coil. A path, in which the operating wire 22 is inserted, is formed in the insertion path component 20.

The torque transmitting sleeve 21 externally mounted on the insertion path component 20 includes a member obtained by embedding a mesh tube 21a, which is formed by netting thin metal wires, in a resin tube 21b. A resin constituting the resin tube 21b is soaked into the mesh of the mesh tube 21a. Both of the front and rear surfaces of the tube 21b are covered with a resin so as to prevent the mesh tube 21a from being exposed. The torque transmitting sleeve 21 can be formed by putting, for example, a mesh tube into between two resin tubes, that is, inner and outer synthetic resin tubes and also applying pressure to these tubes while heating. Alternatively, the torque transmitting sleeve 21 can be formed by molding a molten resin integrally with the mesh tube into a tube-like configuration.

Thus, in the double tube structure, the entire insertion path component 20 serving as an inner layer, and the entire torque transmitting sleeve 21 serving as an outer layer are stacked by bringing the most part of in a lengthwise direction of each of the entire component 20 and the entire sleeve 21 into a non-fixed state. Coupling portions provided at both end portions of each of the insertion path component 20 and the torque transmitting sleeve 21, which respectively correspond to the mounting part 10 of the forceps operation portion 1 and the shaft portion 30 of the operating member 3, are respectively fixed to those provided at both end portions of the other of the insertion path component 20 and the torque transmitting sleeve 21. Accordingly, when the double tube including the insertion path component 20 and the torque transmitting sleeve 21 is bent, the inner layer and the outer layer can slide with respect to each other.

The flexible cord 2 to be provided between the forceps operation portion 1 and the operating member 3 is constituted, as described above. Consequently, the insertion path component 20 formed of the closely-wound coil is configured so that an insertion path, in which the operating wire 22 is inserted, can be assured. Even when pressurized from the outside, or even when the curved part 43b of the insertion portion 43 is bent at maximum in a state in which the flexible cord 2 is inserted in the treatment tool inserting channel 44, neither buckling not deformation does not occur. The shape retention of the insertion path formed in the component 20 can sufficiently be achieved. When the operatingmember 3 is operated, the operating wire 22 inserted in the flexible cord 2 smoothly operates, so that the forceps portions 12, 12 can smoothly be opened and closed. The closely-wound coil constituting the insertion path component 20 has a single layer structure. The torque transmitting sleeve 21 constituting the outer layer can be constituted as a thin member obtained by embedding the mesh tube 21a, which is formed of thin metal wires, in the resin tube 21b. Consequently, reduction in the diameter of the flexible cord 2 of the forceps 4 can be achieved.

To control the directions of the forceps portions 12, 12 in the forceps operation portions 1 in a state, in which the forceps 4 is drawn out of an end of the insertion portion 43, a part of the flexible cord 2 or the operation member 3, which is positioned outer than the treatment tool introducing portion 42, is turned around an axis by being twisted. The flexible cord 2 includes the torque transmitting sleeve 21 formed of the mesh tube 21a embedded in the resin tube 21b. Thus, when torque is applied to the flexible cord 2 from the treatment tool introducing portion 42, the flexible cord 2 is turned around an axis thereof in the treatment tool inserting channel 44, so that the forceps operation portion 1 provided at the flexible cord 2 is turned a desired angle. This torque is accurately transmitted by the torque transmitting sleeve 21. Moreover, the response of torque transmission is good. The insertion path component 20 formed of the closely-wound coil is disposed as the inner layer of the torque transmitting sleeve 21. This closely-wound coil has play. As described above, the response of torque transmission in the torque transmitting sleeve 21 is high. Also, the insertion path component 20 is not firmly fixed to the torque transmitting sleeve 21. Consequently, no torque acts on the insertion path component 20.

Moreover, both the insertion path component 20 and the torque transmitting sleeve 21 of the flexible cord 2 have flexibility in a bending direction. Accordingly, the flexible cord 2 can freely be bent. In addition, because the insertion path component 20 and the torque transmitting sleeve 21 of the double tube structure are not firmly fixed to each other, the resistance to bending of the flexible cord 2 does not act on both of the insertion path component 20 and the torque transmitting sleeve 21. Generally, the closely-wound coil, which is a single layer and constitutes the insertion path component 20, is smaller in the resistance to bending than the torque transmitting sleeve 21. Thus, substantially only a force due to the resistance to bending of one of the insertion path component 20 and the torque transmitting sleeve 21, which is larger in the resistance to bending than the other, that is, only a force due to the resistance to bending of the torque transmitting sleeve 21 acts. Consequently, even in a state in which the curved portion 43b of the insertion portion 43 of the endoscope 40 is largely bent as illustrated in FIG. 5, the forceps 4 can smoothly be inserted in the treatment tool inserting channel 44, so that the rigid tip end portion 43c can smoothly be drawn out therefrom. Accordingly, good operability of inserting the forceps 4 in the treatment tool inserting channel 44 can be obtained.

Additionally, in the case of the flexible cord 2, the torque transmitting sleeve 21 including the resin tube 21b is exposed to the outside. Thus, the flexible cord 2 can be further smoothly inserted into the treatment tool inserting channel 44 by forming the resin tube 21b of a member of good slidability. Also, when the flexible cord 2 is bent, the resin tube 21b and the insertion path component 20 can smoothly slide with respect to each other. Consequently, resistance to bending is further reduced.

The flexible cord of the treatment tool according to the invention has favorable flexibility in a bending direction, sufficient buckling resistance, and excellent flexibility. When the treatment tool is inserted in the bent treatment tool inserting channel, the treatment tool can easily be inserted therein. In addition, the treatment tool can exhibit good operability.

## Claims

1. An endoscopic treatment tool (4) configured to be inserted in an endoscopic-treatment-tool inserting channel (44), the endoscopic treatment tool (4) comprising:
a flexible cord (2);
an operation portion (1) provided at a tip end of the flexible cord (2);
an operating wire (22), inserted in the flexible cord (2), that operates the operation portion (1); and
an operating section (3), provided at a base end portion of the flexible cord (2), that operates the operating wire (22),
wherein the flexible cord (2) comprises a double-tube-like member including an insertion path component (20) and a torque transmitting sleeve (21),
the insertion path component (20) includes a closely wound coil, and the torque transmitting sleeve (21) includes a resin tube (21b) in which a mesh tube (21a) is embedded; and
the insertion path component (20) is configured to be inserted into the torque transmitting sleeve (21) so as to be able to perform a relative sliding motion with respect to the torque transmitting sleeve (21), and the operating wire (22) is configured to be inserted in the insertion path component (20),
**characterized in that** the flexible cord (2) comprises the double-tube-like member over its entire length and that the insertion path component (20) and the torque transmitting sleeve (21) are in a non-fixed state in a lengthwise direction between the tip end and the base end of the flexible cord (2).

## Patentansprüche

1. Endoskopisches Behandlungswerkzeug (4), welches eingerichtet ist, in einen Einführkanal (44) für ein endoskopisches Behandlungswerkzeug eingeführt zu werden, wobei das endoskopische Behandlungswerkzeug (4) umfasst:
eine flexible Leitungsschnur (2);
einen Arbeitsbereich (1), der an einem Kopfende der flexiblen Leitungsschnur (2) vorgesehen ist;
einen Arbeitsdraht (22), der in die flexible Leitungsschnur (2) eingeführt ist und den Arbeitsbereich (1) betätigt; und
einen Arbeitsabschnitt (3), der an einem Basisendabschnitt der flexiblen Leitungsschnur (2) vorgesehen ist und den Arbeitsdraht (22) betätigt,
wobei die flexible Leitungsschnur (2) ein Doppelrohr-artiges Bauteil umfasst, umfassend eine Einführpfad-Komponente (20) und eine Drehmoment-Übertragungshülse (21),
die Einführpfad-Komponente (20) eine eng gewickelte Wicklung umfasst und die Drehmoment-Übertragungshülse (21) ein Harzrohr (21b), in das ein Maschenrohr (21a) eingebettet ist, umfasst; und
die Einführpfad-Komponente (20) eingerichtet ist, in die Drehmoment-Übertragungshülse (21) eingesetzt zu werden, so dass sie eine relative Gleitbewegung bezüglich der Drehmoment-Übertragungshülse (21) ausführen kann und der Arbeitsdraht (22) eingerichtet ist, in die Einführpfad-Komponente (20) eingeführt zu werden,
**dadurch gekennzeichnet, dass** die flexible Leitungsschnur (2) das Doppelrohr-artige Bauteil über seine gesamte Länge umfasst und dass die Einführpfad-Komponente (20) und die Drehmoment-Übertragungshülse (21) in einer Längsrichtung zwischen dem Kopfende und dem Basisende der flexiblen Leitungsschnur (2) in einem nicht fixierten Zustand sind.

## Revendications

1. Outil de traitement endoscopique (4) configuré pour être inséré dans un canal d'insertion pour outil de traitement endoscopique (44), l'outil de traitement endoscopique (14) comprenant :
un cordon souple (2) ;
une portion de manoeuvre (1) prévue au niveau d'une extrémité de tête du cordon souple (2) ;
un fil de manoeuvre (22), inséré dans le cordon souple (2), manoeuvrant la portion de manoeuvre (1), et
une partie de manoeuvre (3), prévue au niveau d'une portion d'extrémité de base du cordon souple (2), manoeuvrant le fil de manoeuvre (22),
dans lequel le cordon flexible (2) comprend un élément similaire à un double tube, incluant un composant de trajet d'insertion (20) et un manchon de transmission de couple (21),
le composant de trajet d'insertion (20) inclut une bobine à enroulement serré, et le manchon de transmission de couple (21) inclut un tube en résine (21b) dans lequel est noyé un tube à mailles (21a), et
le composant de trajet d'insertion (20) est configuré pour être inséré dans le manchon de transmission de couple (21) afin de réaliser un mouvement de glissement relatif par rapport au manchon de transmission de couple (21), et le fil de manoeuvre (22) est configuré pour être inséré dans le composant de trajet d'insertion (20),
**caractérisé en ce que** le cordon souple (2) comprend l'élément similaire à un double tube sur toute sa longueur, et **en ce que** le composant de trajet d'insertion (20) ainsi que le manchon de transmission de couple (21) se trouvent dans un état non fixé dans une direction longitudinale entre l'extrémité de tête et l'extrémité de base du cordon souple (2).
